# EUROPEAN PATENT SPECIFICATION

(11) **EP 1 536 844 B1**
(45) Date of publication and mention of the grant of the patent: **04.03.2009**
(21) Application number: 03749396.2
(22) Date of filing: 02.09.2003
(51) Int. Cl.: A61K 51/04, A61K 51/12, C07F 1/08, C07F 13/00, C07F 15/00

(54) **PREPARATION OF M(CO)3-COMPLEXES BY SOLID PHASE TECHNIQUES VIA METAL ASSISTED CLEAVAGE FROM THE SOLID SUPPORT**
HERSTELLUNG VON M(CO)3-KOMPLEXEN DURCH FESTPHASENTECHNIKEN DURCH METAL-UNTERSTÜTZTE TRENNUNG VOM FESTEN TRÄGER
PREPARATION DE COMPLEXES M(CO) SB 3 /SB PAR DES TECHNIQUES DE PHASE SOLIDE AU MOYEN DU CLIVAGE METALLIQUE DEPUIS LE SUPPORT SOLIDE

(30) Priority: 03.09.2002 EP 02078743
(43) Date of publication of application: 08.06.2005
(73) Proprietor: UNIVERSITY OF ZURICH, 8006 Zürich (CH)
(72) Inventor: ALBERTO, Roger, CH-8400 Winterthur (CH); MUNDWILER, Stefan, CH-5000 Aarau (CH)
(74) Representative: Jones, Nicholas Andrew
(86) International application number: PCT/US2003/027665
(87) International publication number: WO 2004/022105

(56) References cited:
- EP-A- 1 013 642
- WO-A-01/00637
- WO-A-01/25243
- WO-A-01/89586
- WO-A-98/48848
- US-A- 5 789 555
- SCHIBLI R ET AL: "Steps toward high specific activity labelling of biomolecules for therapeutic application: preparation of precursor [[188]Re(H2O)3(CO)3]+ and synthesis of tailor-made bifunctional ligand systems" BIOCONJUGATE CHEMISTRY, AMERICAN CHEMICAL SOCIETY, WASHINGTON, DC, US, vol. 13, no. 4, 7 July 2002 (2002-07-07), pages 750-756, XP002218738 ISSN: 1043-1802
- LA BELLA R ET AL: "A [99m]Tc(I)-postlabelled high affinity bombesin analogue as a potential tumor imaging agent" BIOCONJUGATE CHEMISTRY, AMERICAN CHEMICAL SOCIETY, WASHINGTON, DC, US, vol. 13, no. 3, 15 May 2002 (2002-05-15), pages 599-604, XP002218739 ISSN: 1043-1802
- JANG B-S ET AL: "Synthesis of [99m]Tc-tricarbonyl precursors for labelling of bioactive molecules" JOURNAL OF THE KOREAN NUCLEAR SOCIETY, vol. 34, no. 2, April 2002 (2002-04), pages 146-153, XP001117823 ISSN: 0372-7327
- LANGER M ET AL: "[99m]Tc-labelled neuropeptide Y analogues as potential tumour imaging agents" BIOCONJUGATE CHEMISTRY, AMERICAN CHEMICAL SOCIETY, WASHINGTON, DC, US, vol. 12, November 2001 (2001-11), pages 1028-1034, XP001059452 ISSN: 1043-1802
- SEIFERT S ET AL: "REACTIVITY OF TECHNETIUM(I) THIOETHER CARBONYL COMPLEXES TOWARDS HISTIDINE-AN EXAFS STUDY IN SOLUTION" INORGANICA CHIMICA ACTA, LAUSANNE, CH, vol. 322, 8 October 2001 (2001-10-08), pages 79-86, XP001074703 ISSN: 0020-1693
- CORREIA J D G ET AL: "Re Tricarbonyl Complexes with Ligands Containing P,N,N and P,N,O Donor Atom Sets: Synthesis and Structural Characterisation" INORGANIC CHEMISTRY, AMERICAN CHEMICAL SOCIETY, WASHINGTON, DC, US, vol. 40, no. 20, 24 September 2001 (2001-09-24), pages 5147-65151, XP002218740 ISSN: 0020-1669
- PETRIG J ET AL: "Derivatisation of glucose and 2-deoxyglucose for transition metal complexation: substitution reactions with organometallic [99m]Tc and Re precursors and fundamental NMR investigations" CHEMISTRY - A EUROPEAN JOURNAL, WILEY-VCH, WEILHEIM, DE, vol. 7, no. 9, 4 May 2001 (2001-05-04), pages 1866-1873, XP002218741 ISSN: 0947-6539
- FALLER J W ET AL: "The synthesis of new complexes of rhenium(I) with heterotridentate [P,N,O] ligands" JOURNAL OF ORGANOMETALLIC CHEMISTRY, ELSEVIER-SEQUOIA S.A. LAUSANNE, CH, vol. 626, no. 1-2, 30 April 2001 (2001-04-30), pages 181-185, XP004235661 ISSN: 0022-328X
- SCHIBLI R ET AL: "Influence of the denticity of ligand systems on the in vitro and in vivo behaviour of [99m]Tc(I)-tricarbonyl complexes: a hint for the future functionalisation of biomolecules" BIOCONJUGATE CHEMISTRY, AMERICAN CHEMICAL SOCIETY, WASHINGTON, DC, US, vol. 11, no. 3, May 2000 (2000-05), pages 345-351, XP002218742 ISSN: 1043-1802
- POLLAK A ET AL: "A convenient method of preparing high specific activity technetium complexes using thiol-containing chelators absorbed on gold" JOURNAL OF THE AMERICAN CHEMICAL SOCIETY, AMERICAN CHEMICAL SOCIETY, WASHINGTON, DC, US, vol. 121, no. 49, 15 December 1999 (1999-12-15), pages 11593-11594, XP002218743 ISSN: 0002-7863 cited in the application
- ALBERTO ET AL: "A Novel Organometallic Aqua Complex of Technetium for the Labeling of Biomolecules: Synthesis of [99mTc(OH2)3(CO)3]+ from [99mTcO4]- in Aqueous Solution and Its Reaction with a Bifunctional Ligand" JOURNAL OF THE AMERICAN CHEMICAL SOCIETY, AMERICAN CHEMICAL SOCIETY, WASHINGTON, DC, US, vol. 120, no. 31, 12 August 1998 (1998-08-12), pages 7987-7988, XP002149527 ISSN: 0002-7863
- STOR G J ET AL: "Spectroelectrochemical (IR, UV/Vis) determination of the reductive pathways for a series of [Re(CO)3(alpha-diimine)L']0/+ (L' = Halide, Otf-, THF, MeCN, n-PrCN, PPh3, P(OMe)3) complexes" ORGANOMETALLICS, AMERICAN CHEMICAL SOCIETY, WASHINGTON, DC, US, vol. 14, no. 3, March 1995 (1995-03), pages 1115-1131, XP002218744 ISSN: 0276-7333
- ALBERTO R ET AL: "New organometallic technetium complexes in high and low oxidation states" RADIOCHIMICA ACTA, LONDON, GB, vol. 63, 1993, pages 153-161, XP002060831
- DUNN-DUFAULT R ET AL: "A solid-phase technique for preparation of no-carrier-added technetium-99m radiopharmaceuticals: application to the streptavidin/biotin system" NUCLEAR MEDICINE AND BIOLOGY, ELSEVIER SCIENCE PUBLISHERS, NEW YORK, NY, US, vol. 27, no. 8, November 2000 (2000-11), pages 803-807, XP004228484 ISSN: 0969-8051 cited in the application
- KHEHYONG N ET AL: "Preparation of Acid-labile Resins with Halide Linkers and their Utility in Solid Phase Organic Synthesis" TETRAHEDRON LETTERS, ELSEVIER SCIENCE PUBLISHERS, AMSTERDAM, NL, vol. 38, no. 6, 10 February 1997 (1997-02-10), pages 973-976, XP004033909 ISSN: 0040-4039 cited in the application
- VAN WIJNKOOP M ET AL: "1,3-Dipolar cycloaddition reactions to the C=X-M fragment. 7. Reaction of Ru(CO)3(i-Pr-DAB) with dimethyl acetylenedicarboxylate. X-ray crystal structure of the protonated initial bicyclo[2.2.1] adduct" ORGANOMETALLICS, AMERICAN CHEMICAL SOCIETY, WASHINGTON, DC, US, vol. 11, no. 11, November 1992 (1992-11), pages 3607-3617, XP002218745 ISSN: 0276-7333

## Description

The invention relates to the field of radiopharmaceuticals. In particular the invention relates to a process for the preparation of a metal complexed agent via metal assisted cleavage from a solid support.

In a further aspect the invention relates to new solid phase bound conjugates of a ligand and a biomolecule.

In yet a further aspect the invention relates to new metal complexed ligand-biomolecule conjugates, compositions comprising these new complexes and their use.

In still a further aspect the invention relates to a kit for the preparation of a diagnostic or therapeutic pharmaceutical composition.

For the application of radiolabeled bioactive molecules such as i.e. peptides in clinical routine diagnosis or therapy it is highly desirable that only labeled compounds are injected to avoid saturation of the corresponding receptors *in vivo* or toxic side effects from "cold", unlabeled compounds. Furthermore, binding of large amounts of unlabeled biomolecules to the receptors spoils the possibility of getting clear images (scintigrams) and, thus, often disables a clear diagnosis.

According to the state of the art, high specific activity in a normal homogenous labeling procedure can only be achieved by using the lowest possible amount (concentration) of derivatized biomolecules (or ligand for ^{99m}Tc which is coupled to the biomolecule) which still results in quantitative labeling. Depending on the ligand and the complex precursor, these amounts often have to be relatively high since at low concentrations the rate of complexation is governed by a second order kinetic and, thus, labeling is too slow and accompanied by decomposition of ligand or ^{99m}Tc precursor. The lowest concentration limit is often not convenient in routine use, since slightly changed conditions (temperature, time) at such a concentration do not end up with quantitative labeling yield. Correspondingly, side- and decomposition products as well as starting materials are still present in the final solution.

A convenient way of a physically separating 'cold' from 'hot' compound is by attaching the ligand-biomolecule conjugate to a solid phase material and cleave it from there concomitantly with the complex formation. Examples for such metal assisted cleavage from solid phases are rare.

American patent US-5,789,555 (Pollak et al.) describes a process for labeling peptides with technetium-99m, rhenium-186 or rhenium-188. The process comprising the steps of covalently coupling the peptides to a solid support, by means of a thioether bond with a maleimide linker. By introducing pertechnetate to the support, a ^{99m}Tc^{V}(=O)-peptide complex is formed. Upon complex formation, ^{99m}Tc^{V}(=O) catalyzes cleavage of the peptide from the support, by breaking the C-S bond, thus releasing the ^{99m}Tc^{V}(=O)-peptide complex from the support.

It is known from literature, that protected thiols release the protecting group by coordination to a Tc=O center. Based on these findings Pollak et. al. (J. Am. Chem. Soc. 121, 11593-11594 (1999) bound a tetradentate chelator via a thioether bond to a gold surface. Upon coordination of Tc(V) to this ligand the ^{99m}Tc-complex was selectively released into solution by breaking the S-Au-bond as the sulfur coordinated to the Tc.

More recently, Dunn-Dufault, et. al. (Nucl. Med. Biol. 27, 803-807 (2000)) described a variant of this method by covalently binding the chelator to an organic polymeric support.

The above mentioned processes for producing Tc and Re labeled organic complexes all depend on cleavage of a C-S or Au-S bond. This C-S and Au-S bond, with which the ligand is covalently bound to the solid support, is sensitive to oxidation. Therefore, it is necessary to store solid supports comprising ligands covalently linked via a C-S bond under reducing conditions. This is especially true for long term storage. The necessity of storage under reducing conditions requires additional measures to be taken for storage. Moreover, if the supports are to be used for the generation of compounds suitable for pharmaceutical applications, the presence of reducing agents is highly undesirable from the standpoint of pharmaceutical safety. Therefore, there will be certain restrictions for use of the known solid bound ligands for such application.

Additionally, the use of these metal oxide species is accompanied by restrictions to the ligands that are available for use therewith i.e. tetradentates. Hence the sole disclosure in the prior art of peptidic ligands for use with a ^{99m}Tc^{V}(=O) center.

Schibli et al. (Bioconjugate Chem. 11,3445 (2000)) describe the synthesis in solution of a number of multidentate complexes using [^{99m}Tc (OH₂)₃(CO) ₃]⁺. Schibli et al (Bioconjugate Chem. 13,750(2002)) describe the solution phase synthesis of multidentate complexes using [¹⁸⁸Re(H₂O)₃(CO)₃]⁺. Seifert et al. (Inorg. Chim. Acta 322,79(2001)) disclose the solution phase synthesis of Tc(I) thioether carbonyl complexes and the solution phase ligand exchange reaction thereof with histidine. La Bella et al. (Bioconjugate Chem. 13,599 (2002)) describe the preparation of a bombesin analogue which is labelled, in solution, using [^{99m}Tc(OH₂)₃(CO)₃]⁺. Jang et al. (J. Korean Nucl. Soc. 34, 146 (2002)) describe the radiolabelling of a number of biomolecules, in solution, using [^{99m}Tc(OH₂)₃(CO)₃]⁺. Langer et al. (Bioconjugate Chem. 12,1028 (2001)) have disclosed a similar approach to the preparation, in solution, of radiolabelled derivatives of neuropeptide Y. Synthesis of rhenium tricarbonyl complexes in solution using the precursor (NEt₄)₂[ReBr₃(CO)₃] is described by Correia et al. (Inorg. Chem. 40,5147 (2001)). Labelled sugar derivatives prepared in solution using [M(H₂O)₃(CO)₃]⁺ (M=Tc,Re) have been described by Petrig et al. (Chem. Eur. J. 7,1868 (2001)). Preparation of rhenium complexes with phosphorus-containing tridentate ligands using Re(CO)₅ Br in solution is described by Faller et al. (J. Organometallic Chem. 626,181 (2001)). Solution phase reaction of [^{99m}Tc(OH₂)₃(CO)₃]⁺ with a bifunctional tridentate ligand is also disclosed in Alberto et al. (J. Am. Chem. Soc. 120, 7987 (1998)). The solution phase syntheses of a series of Re(CO)₃ diimine-containing complexes is described by Stor et al. (Organometallics 14,1115 (1995)). Solution phase syntheses of Tc(CO)₃ - containing complexes are also disclosed in Alberto et al. (Radiochimica Acta 63,153 (1993)). Solution phase preparation of complexes using a precursor based on Ru(CO)₃ is disclosed by van Wijnkoop et al. (Organometallics 11,3607 (1992)).

WO01/00637 discloses the solution phase syntheses of a number of Group (VII) transition metal complexes using the [M(CO)₃(OH)₂)₃]⁺ precursor, together with a kit for carrying out the syntheses. Similar subject matter is disclosed in WO01/89586. Tc(CO)₃ and Re(CO)₃ complexes, prepared from solution, are also disclosed in EP1013642.

The solid phase derivatisation of a biomolecule is described in Kheyong et al. (Tetrahedron Lett. 38,973 (1997)). However, the cleavage of the biomolecule from the solid support does not involve a metal and is a non-specific step requiring merely the presence of acid.

Thus there is a need for new processes for preparing metal labeled complexes by solid phase techniques via metal assisted cleavage from the solid support which employ solid phase bound biomolecule-ligand conjugates which are more stable under pharmaceutically acceptable conditions than the prior art conjugates.

Additionally, the availability of more ligands that can be used in the formation of metal complexed ligand-biomolecule conjugates by means of solid phase techniques via metal assisted cleavage will be advantageous, since this will provide a more flexible use of this technique. It is the object of the present invention to provide improved techniques for the preparation of labeled diagnostic and therapeutic compounds.

In the research that led to the present invention, it was found that some organic molecules (ligands) that are able to coordinate to a metal and bound to a solid support via a tertiary amine group, in the presence of [Tc(H₂O)₃(CO)₃]⁺, cleave from the solid support upon formation of [Tc(CO)₃-Ligand]-complexes. The selective hydrolytic C-N bond cleavage is clearly mediated by the low valent carbonyl [Tc(H₂O)(CO)₃]⁺ center, formed during complex formation, and does not occur under the same reaction conditions in the absence of [^{99m}Tc(H₂O)₃(CO)₃]⁺. After release from the solid support, the former tertiary amine is present as a coordinated secondary amine.

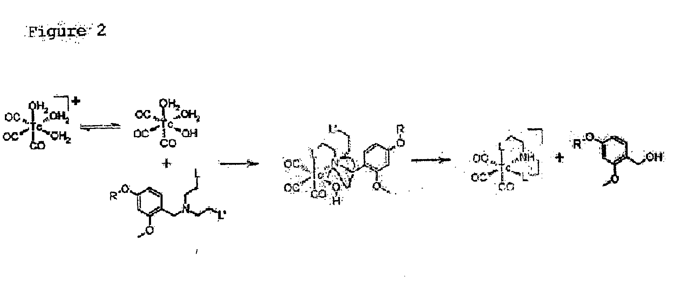

The mechanism for this [Tc(CO)₃-Ligand]-complex is proposed to be as follows. As the tertiary amino group of the solid-bound chelator (the so-called ligand) coordinates to the cationic metal center of [M(H₂O)₃(CO)₃]ⁿ⁺, it becomes partially positive and the adjacent carbon atom is therefore activated for nucleophilic attack. A remaining hydroxy group attacks the methylene group of the chelator and induces C-N bond cleavage (Figure 2). The third donor site of the chelator coordinates to the metal center, and the product complex is released into solution. Uncomplexed chelator and uncleaved complex remain bound to the solid phase.

It was found that labeled compounds obtained by hydrolytic cleavage of the ligand from the solid support with [^{99m}Tc(H₂O)₃(CO)₃]⁺ as described above had a very high specific activity i.e. there was little uncomplexed ligand in solution. The amount of uncomplexed ligand in solution was in the order of 10⁻⁷ M. Therefore, this specific cleavage reaction can be attractively exploited for the preparation of so-called "no carrier added" (n.c.a.) complexes of technetium and other metals with a similar chemical reactivity.

Thus the invention relates to a new process for generating a metal complexed agent, comprising contacting (I) a solid phase bound organic conjugate represented by the formula 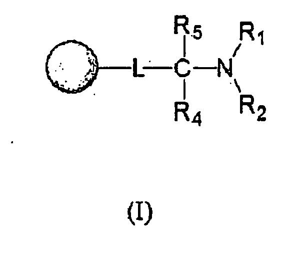 wherein:
the sphere is the solid phase;
C is a methylene group; R4 and R5 are independently selected from the group consisting of H, aliphatic or aromatic substituents, RO, RS and R₂N, wherein R is an aliphatic or arylic group;
L is a linker or a single bond, the linker, when present, being coupled to the solid support and having activating properties towards nucleophilic attack to the C group; and
R1 and R2 are the same or different and are a metal coordinating group or a non-coordinating organic group,
a metal coordinating group derivatized with a biologically active molecule, or a non-coordinating organic group derivatized with a biologically active molecule,
with (II) [M(H₂O)3(CO)₃]ⁿ⁺,
wherein M is selected from the group consisting of technetium (Tc), rhenium (Re), rhodium (Rh), ruthenium (Ru), platinum (Pt), iridium (Ir) and copper (Cu) and n is 1, 2 or 3 depending on the metal; under suitable conditions to cause the formation of a coordinate bond between [M(H₂O)3(CO)₃]ⁿ⁺ and the tertiary amine nitrogen atom of the solid phase bound organic conjugate and thereby the release of the metal complexed agent thus formed from the support.

The linker may or may not be present. It may be already present in the available solid support or can be introduced later. When it is present it is preferably a good activating group for nucleophilic attack at C and selected from the group consisting of phenyl, vinyl, aryl and other non-aliphatic or aliphatic groups. The phenyl, vinyl, aryl, other non-aliphatic or aliphatic group may be substituted, and if they are they are preferably substituted with an electron withdrawing group selected from OR, R, NR₂, wherein R is an aliphatic or an arylic group.

In a preferred embodiment the linker is as shown in formula II: 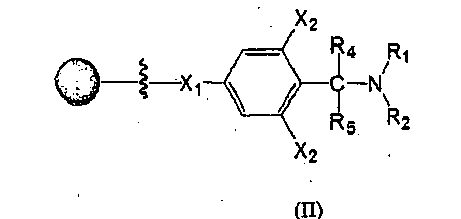 wherein X1 is O and each X2 is H or an electron withdrawing substituent, provided that at least one X2 group is an electron withdrawing substituent, and preferably a -OCH₃ group.

When R1 and R2 or one of R1 and R2 are non-coordinating organic groups they may be selected from alkyl, phenyl or benzyl or derivatives thereof.

Preferably, R1 and/or R2 are selected from the group consisting of

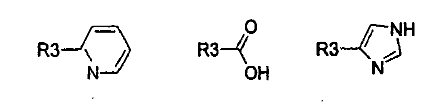

R3-NH₂

wherein R3 is an aliphatic chain containing between 1 and 3 carbons.

The metal M is selected from the group consisting of Tc, Re, Ru, Rh, Ir, Cu and Pt. The metal is most preferably ^{99m}Tc, ¹⁸⁶Re or ¹⁸⁸Re.

Preferably the metal is suitable for use as an imaging agent, e.g. by transmission of high-energy particles or paramagnetic characteristics, or as a radionuclide.

[M(H₂O)₃(CO)₃]ⁿ⁺ can be generated by any suitable means known in the art. Suitable means for generating [M(H₂O)₃(CO)₃]ⁿ⁺ are for example from the permetallate form as disclosed by Alberto et al. (J. Am. Chem. Soc. 123, 3135-3136 (2001)) or in WO98/48848 (Alberto et al.).

The molecule according to formula I without the solid support is called herein the ligand. The ligand can be in particular a tridentate ligand if R1 and R2 are selected from the group consisting of

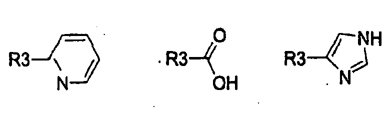

R3-NH₂

but also a bidentate chelator if R1 and R2 or one of both are an aliphatic or aromatic non-coordinating group.

The ligands used according to the invention in combination with [M(H₂O)₃(CO)₃]ⁿ⁺ can be a diversity of tridentate ligands, the main requirement being the presence of a tertiary amine group as the central part of the ligand, which forms the C-N bond that is coupled with the solid support and cleaved upon complex formation. Preferably the ligands used are those based on aliphatic or aromatic amines or carboxylates and combinations thereof as donors. The ligand can also be a bidentate chelator if R1 and R2 but not both is a non-coordinating aliphatic or aromatic group. This is shown in Example 10.

In particular diethylene triamine, picolylamine-*N-*acetic acid, *N*-(2-aminoethyl)-glycine or imino-diacetic acid can be used as ligands in the invention.

The ligand can be covalently linked to the solid support by first forming a halogenated resin e.g. by the methods described by Ngu and Patel (Tet. Lett. 38, 973 - 976 (1997). This halogenated resin can subsequently be reacted with a protected ligand. After deprotection, the ligand bound resin is obtained. If the ligand is attached to the solid phase by this method the covalent bond attaching it to the solid support will be a C-N bond. The ligand can also be synthesized on the solid support starting from an amino resin, e.g. by reductive amination and/or alkylation with alkyl halides as described in examples 5, 6, 8 and 9. Preparation of a loaded resin is discussed in more detail in examples 1-9.

In this specification the term ligand refers to a compound comprising at least one metal coordinating atom capable of forming a coordinating bond with a metal to form a stable metal-ligand complex. A ligand comprising more than one metal coordinating atom may be referred to as a chelator or a multidentate ligand. Bidentate ligands are ligands with two metal-coordinating atoms, tridentate ligands are ligands with three metal-coordinating atoms and tetradentate ligands are ligands with four metal coordinating atoms.

The biologically active molecule (also called herein "biomolecule") that may be coupled to the ligand can be any molecule that is active in diagnosis or therapy. The molecule can be coupled at any position except at the nitrogen linked to the solid support. It may be a targeting molecule for directing the radioactive product to the site that needs to be diagnosed or treated or it may have a therapeutic activity that is independent from the radiolabel. The biologically active molecule may be selected from the group consisting of amino acids; steroids; proteins, in particular structural proteins, enzymes or antibodies; carbohydrates; polysaccharides and oligosaccharides; nucleosides, nucleotides, oligonucleotides and polynucleotides; lipids, peptides and pharmaceutically active small molecules such as central nervous system receptor binding compounds.

The biomolecule can be linked to the ligand with any suitable means known in the art e.g. by reductive amination of an aldehyde to a primary amine group of the ligand or by introducing a binding site at the arylic system. The biomolecule can be linked to the ligand prior to or after binding the ligand to the solid support.

It was found that the choice of the solid support may further improve the efficiency of the process of the invention. The solid support has to be able to swell in water, it has to be stable at reaction conditions, and it must not contain metal coordinating units. This is in particular the case when the solid support is a polyethylene glycol resin, or a hybrid of polyethylene glycol and polystyrene, e.g. a polystyrene resin with polyethylene glycol spacers with a benzyl alcohol anchoring group.

The process of the invention may further comprise the step of collecting the metal complexed agent (i.e. the radiopharmaceutical) for further use.

After preparation of n.c.a. ^{99m}Tc radiopharmaceutical, the solid phase polymer can be collected, washed and reused.

Preferably, the process is performed at a pH that is in the range of about 6.0-11.0, preferably in the range of about 7.5-9.5.

Suitable temperatures for performing the reaction are within the range of about 40-100°C. Preferably the reaction is performed in the range of about 70-82°C.

According to a further aspect thereof the invention relates to the solid phase bound organic conjugate: of formula I, and compositions comprising such a compound. Preferably these compositions are in a form, which can be stored during prolonged time periods under pharmaceutically acceptable conditions.

With the process according to the invention metal complexed ligand-biomolecule conjugates can be obtained with a high specific activity by filtration without further post-labeling purification.

Usually the conjugates obtainable with the process according to the invention are comprised in a composition which is the result of the process of the invention, and which is characterized in that it is essentially free of uncomplexed ligand-biomolecule conjugate e.g. the level of uncomplexed ligand-biomolecule conjugate in the composition containing them is in the 10⁻⁷ M range.

Due to the short half-life of some isotopes used in radiopharmaceuticals, e.g. ^{99m}Tc, labeling of ligand-biomolecule conjugates just prior to their use can be important for the specific activity of the complexed conjugate. The amount of decayed complex in a freshly labeled composition will be lower compared to the situation when the conjugate was complexed a substantial amount of time prior to its use.

Therefore, in yet a further aspect the invention relates to a kit for the preparation of a diagnostic or therapeutic pharmaceutical composition, comprising a container with the molecule of formula (I), in which the reaction with a solution of [M(H₂O)₃(CO)₃]ⁿ⁺ take place. The container can be a vessel or column. The solution of [M(H₂O)₃(CO)₃]ⁿ⁺ is introduced into the vessel or column to start the reaction. The solution can be part of the kit or provided by other means. In an alternative embodiment, the reagents for the preparation of the metal carbonyl [M(H₂O)₃(CO)₃]ⁿ⁺ are comprised in the kit. In addition, the kit may comprise a facility for filtration.

The use of a kit further provides flexibility to the metal complex that can be formed since a selection of a suitable metal can be made just prior to the complexation reaction.

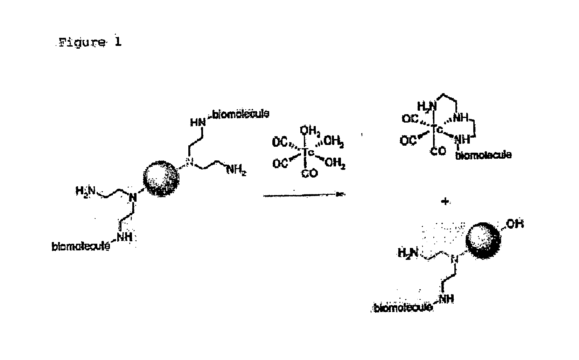

The principle of the preparation of no carrier added (n.c.a.) metal complexed compounds according to the invention is explained in **Figure 1****.** A tridentate ligand e.g. diethylene triamine is bound via a linker, here a benzyl derivative, to a solid phase via a tertiary amine. To the chelator (ligand) a biomolecule, is attached, thus forming a ligand-biomolecule conjugate. Upon introduction of [Tc(H₂O)₃(CO)₃]⁺, complex formation occurs and the tridentate ligand replaces two aqua ligands. The remaining hydroxy ligand on [Tc(H₂O)₂(OH)(CO)₃]⁺ can now attack the activated methylene group to induce C-N-bond cleavage. Activation of the methylene group occurs by complexation of the tertiary amino group to the technetium center, which withdraws electron density from the chelator and make it susceptible for nucleophilic attack.

The main species with reactivity towards the tertiary amine atom of the solid phase bound biomolecule-ligand conjugate is [M (OH) (H₂O)₂(CO)₃]. In solution [M(OH)(H₂O)₂(CO)₃] is in equilibrium with the conjugate in the form of [M(H₂O)₃(CO)₃]ⁿ⁺ and further dissociated forms, depending on the pH of the solution. It will be understood that, depending on the pH, [M(OH)(H₂O)₂(CO)₃] is at least partially interchangeable with these species due to the equilibrium.

The invention is further explained with the following non-restrictive examples. In the Examples reference is made to the following figures:
**Figure 1****:** The principle of the preparation of no carrier added (n.c.a.) metal complexed compounds according to the invention.
**Figure 2****:** Mechanism of complex formation.
**Figure 3****:** pH dependence of the cleavage reaction.
**Figure 4****:** Temperature dependency of cleavage yield.

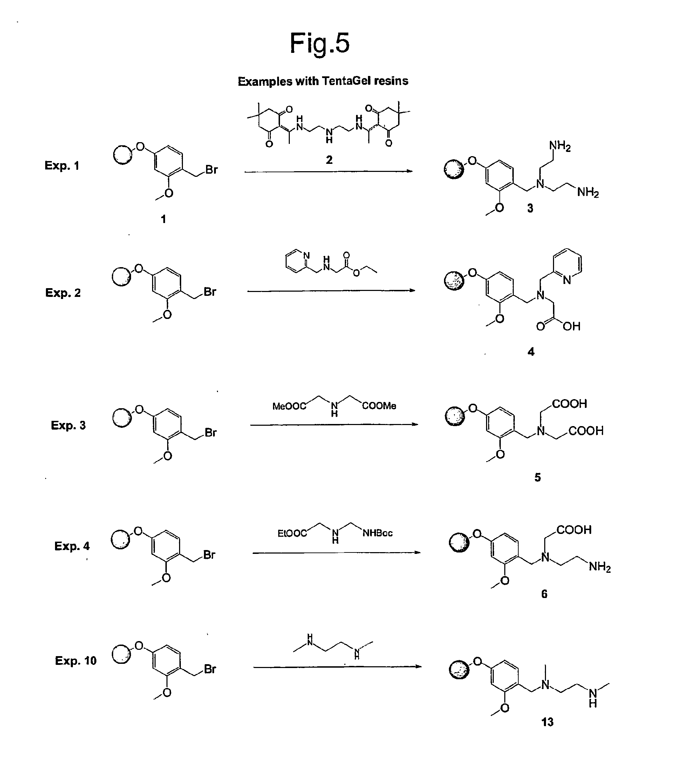

**Figure 5****:** Reaction schemes of Examples 1, 2, 3, 4 and 10.

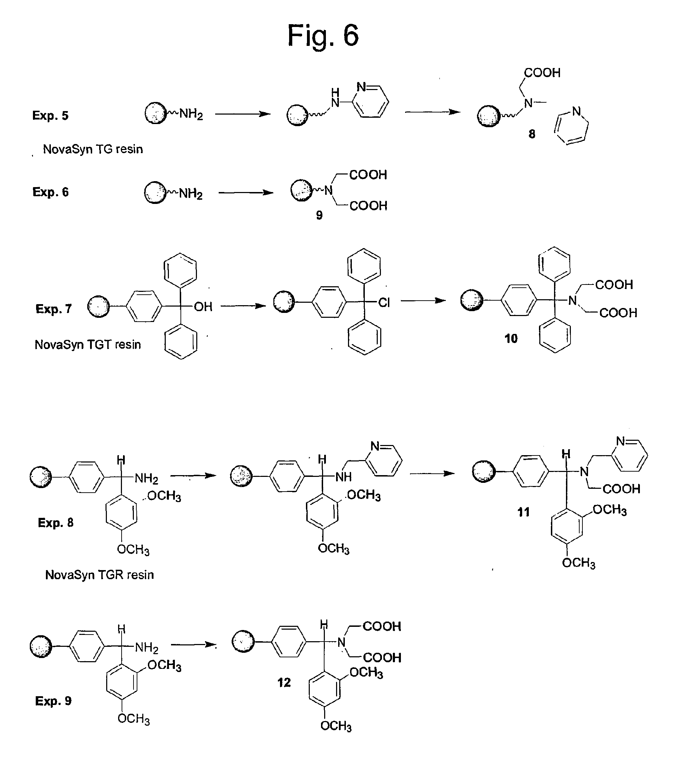

**Figure 6****:** Reaction schemes of Examples 5, 6, 7, 8 and 9.

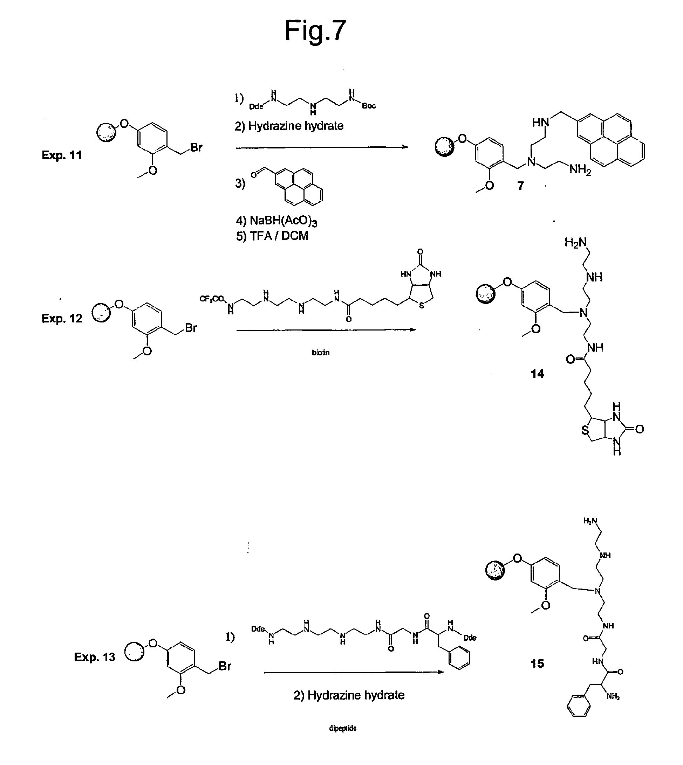

**Figure 7****:** Reaction schemes of Examples 11, 12 and 13.

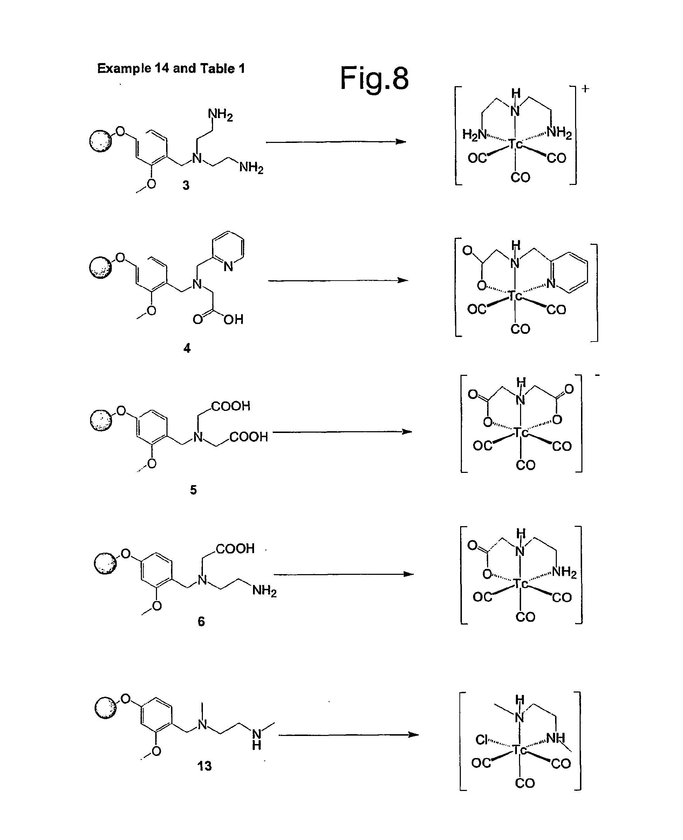

**Figure 8****:** Structural formulas of the compounds of Example 14 and Table 1.

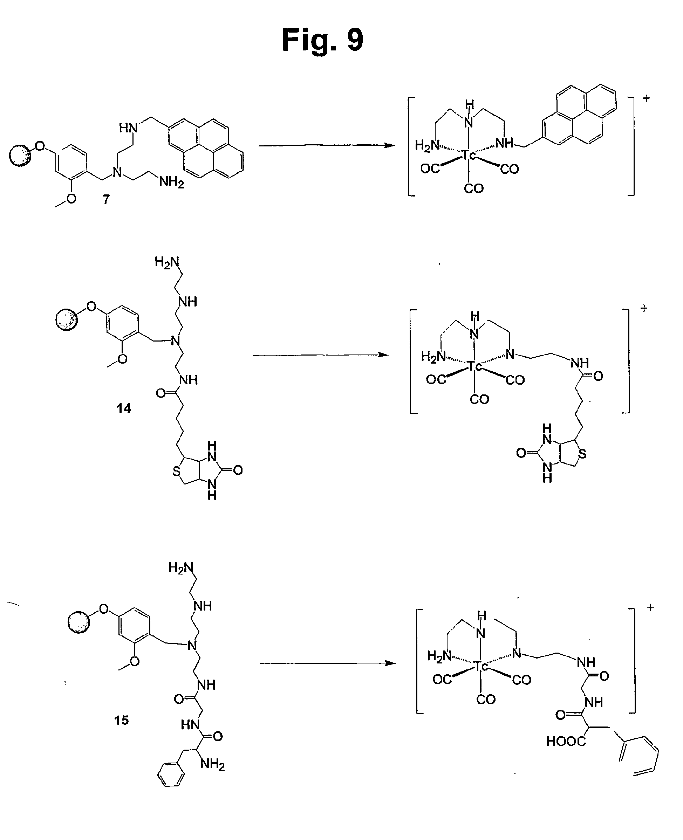

**Figure 9****:** Structural formulas of the compounds with biologically active molecules.

### EXAMPLES

### EXAMPLE 1

A model ligand was covalently attached to an appropriate solid phase resin. The solid phase resin has to swell in water to allow diffusion of the Tc-species, and the anchoring group to which the ligand is coupled has to be an activating group for nucleophilic attack. The polystyrene/polyethylene glycol resin TentaGel S AC (Rapp Polymere GmbH, Tübingen, Germany) fulfills these requirements. Its active site, a benzyl alcohol derivative, was converted into the corresponding bromide 1 (Ngu and Patel, Tet. Lett. 38, 973 - 976 (1997)).

*N*,*N*''-Bis(1-(4,4-dimethyl-2,6-dioxocyclohexyliden) ethyl)diethylenetriamine (2) (101.8 mg, 235.9 µmol) was dissolved in DMF (5 ml), resin 1 (196.6 mg, 47.2 mol) was added, and the mixture was gently stirred at room temperature for 22 hours. The reaction mixture was filtered, and the resin was washed with DMF (3 times), DMF and methanol (3 times alternating), and DMF (5 times). The protecting groups were removed by washing the resin 10 times with a solution of hydrazine hydrate, 2% in DMF (1 ml) for 5 minutes. The reaction mixture was filtered, and the resin was washed with DMF (3 times), DMF and methanol (5 times alternating), DMF (3 times), and diethyl ether (3 times). The resin was dried at high vacuum to give product 3 in a yield of 192.3 mg (97.8%; capacity 0.236 mmol/g, coupling efficiency 100%).

Free amino groups on the solid phase resins were visualized by color tests: bromophenol blue solution in water for alkaline resins and trinitrotoluenesulfonic acid (TNBS) in DMF/diisopropylethylamine 10:1 exclusively for primary amines. Resin 3 was positive in both tests, whereas the protected intermediate was negative on TNBS staining. The capacity of the resins (in mmol of bound chelator per gram) and the efficiency for the coupling of the chelators to the solid supports were calculated from the N-content of the resins as determined by elementary analyses.

The chelating capacity of resin 3 was verified by stirring it in a 1mM solution of [⁹⁹Tc(H₂O)₃(CO)₃]⁺ (7 equivalents) at room temperature. Analyzing of the filtrated solution by β-liquid scintillation counting showed a decrease of activity of 14%, which is consistent with quantitative complex formation on the resin. HPLC analyses exhibited only peaks of the starting material, indicating that no cleavage from the solid phase occurred under these mild conditions. The once formed ⁹⁹Tc-complex turned out to be stable under the conditions used for labeling. Even prolonged heating at 80°C for 5 hours in phosphate buffer pH 7.5, yielded only 3% beta-activity in the solution, at least one order of magnitude lower than expected.

### EXAMPLE 2

An other tridentate ligand, yielding non charged complexes, was attached to the same resin as in Example 1. *N-*Picolylamine acetic acid ethyl ester (54 mg, 280 µmol) was dissolved in DMF (3 ml) and resin 1 (280 mg, 67 µmol) was added. The mixture was gently stirred at room temperature for 15 hours, the reaction mixture was filtered, and the resin was washed with DMF (3 times), DMF and methanol (3 times alternating), and diethyl ether (3 times). The protected intermediate was positive on bromophenol blue and negative on TNBS staining. The protecting groups were removed by gently stirring the resin in a mixture of water (5 ml) and sodium hydroxide 1M (0.30 ml) for 28 hours. Filtration of the resin, washing with DMF (3 times), DMF and methanol (3 times alternating), DMF (3 times), and diethyl ether (3 times) and drying at high vacuum gave product **4** in a yield of 268 mg (96%; capacity 0.209 mmol/g, coupling efficiency 87.1%). Resin **4** was negative on all of the staining reactions.

### EXAMPLE 3

An other tridentate ligand, yielding negatively charged complexes, was attached to the same resin as in example 1. Dimethylimino diacetate hydrochloride (6.4 mg, 33 µmol) and diisopropylethylamine (11.2 µl, 66 µmol) were dissolved in DMF (0.5 ml) and resin 1 (280 mg, 67 µmol) was added. The mixture was gently stirred at room temperature for 24 hours, the reaction mixture was filtered, and the resin was washed with DMF (3 times), DMF and methanol (3 times alternating), methanol and water (3 times alternating). The protected intermediate was positive on bromophenol blue and negative on TNBS staining. The protecting groups were removed rinsing the resin with aqueous NaOH (0.1M for 3 hours, then 0.01M for 12 hours). Filtration of the resin, washing with NaOH 0.1M (2 times), water (5 times), water and methanol (3 times alternating), methanol (3 times), and diethyl ether (3 times) and drying at high vacuum gave product 5 in an yield of 35 mg (100%; capacity 4 mmol/g, coupling efficiency 18%). Resin 5 was negative on all of the staining reactions.

### EXAMPLE 4

Another tridentate ligand, yielding non-charged complexes, was attached to the same resin as in example 1.

N⁵-tert-butyloxycarbonyl)-5-amino-3-azapentane acid ethyl ester(74 mg, 280 µmol) was dissolved in DMF (3 ml) and resin 1 (300 mg, 72 µmol) was added. The mixture was gently stirred at room temperature for 15 hours, the reaction mixture was filtered, and the resin was washed with DMF (3 times), DMF and methanol (3 times alternating), and diethyl ether (3 times). The protected intermediate was positive on bromophenol blue and negative on TNBS staining. The ethyl ester group was removed by gently stirring the resin in a mixture of water (5 ml) and sodium hydroxide 1M (1.40 ml) for 28 hours. Filtration of the resin, washing with DMF (3 times), DMF and methanol (3 times alternating), DMF (3 times), and diethyl ether (3 times) and drying at high vacuum gave the Boc protected acid. The Boc group was removed by stirring the resin in a mixture of TFA and DCM (1:1) for 5 minutes, filtration, and by stirring the resin in a mixture of TFA and DCM (1:1) again, now for 10 minutes. Washing as described above gave product 6 in a yield of 72 mg (96%, capacity 22 mmol/g, coupling efficiency 94%). Resin **6** was positive on TNBS staining.

### EXAMPLE 5

NovaSyn TG resin has an aliphatic amino anchoring group. In this example, the synthesis of the chelator picolylamineacetic acid on the solid support is described.

NovaSyn TG resin (100 mg, 30 umol)and pyridine-2-carbaldehyde (14.3 µl, 150 µmol) were stirred in methanol (3 ml) at room temperature for 20 hours. The reaction mixture was filtered, and the resin was washed with DMF (3 times), and DMF and methanol (3 times alternating). Sodium triacetoxy- borohydride (31.8 mg, 150 µmol) in DMF (2 ml) was added to the resin. After stirring at room temperature for 5 hours, the reaction mixture was filtered, and the resin was washed with DMF (4 times) and methanol (3 times). NaHCO₃ (10% in water) was added to the resin. After 3 hours, the resin was washed with DMF (3 times), water (3 times), ethanol (3 times), and diethyl ether (3 times) and dried to give the aminopyridine intermediate which was positive on bromophenol blue and slightly positive on TNBS staining.

A mixture of bromoacetic acid ethyl ester (16.6 µl, 150 µmol) and diisopropyl ethylamine (5.1 µl, 30 µmol) in ethanol (2.5 ml) was added to the resin. After stirring at room temperature for 24 hours, the reaction mixture was filtered, and the resin was washed with ethanol (5 times) and dried. NaOH (1 M) was added to the resin to remove the ethyl ester protecting group. After one day stirring at room temperature, the reaction mixture was filtered, and the resin was washed with water (5 times), ethanol (3 times) and diethyl ether (2 times) and dried to give **8** in a yield of 86.4 mg (83.7%; capacity 0.10 mmol/g, coupling efficiency 43%).

### EXAMPLE 6

NovaSyn TG resin (100 mg, 30 µmol), ethyl bromoacetate (33.2 µl, 300 µmol) and diisopropylethylamine (12.9 µl, 75 µmol) were reacted as described in the second part of example 5 to give 9 in a yield of 96.2 mg (93%).

### EXAMPLE 7

NovaSyn TGT resin has a hydroxytrityl anchoring group. The hydroxy group was converted to the chloride as described (J.M.J Frechert et al., Tetrahedron Lett. 1975, 3055).

Dimethyliminodiacetate hydrochloride (16.2 mg, 82 µmol) and diisopropylethylamine (21 µl, 123 µmol) were dissolved in DMF (2 ml). Chlorinated Novasyn TGT resin (41 µmol) was added. The coupling reaction as well as the ester hydrolyses were done as described in example 3 with the exception that diisopropylethylamine (14 µl, 82 µmol) was added after 3 hours reaction time. Product **10** was obtained in a yield of 132 mg (81%; capacity 0.015 mmol/g, coupling efficiency 6%).

### EXAMPLE 8

NovaSyn TGR resin has an aminomethyl anchoring group with two aryl substituents on the methylene group. Resin **11** was prepared analogous to the synthesis of **8** described in Example 5.

NovaSyn TGR resin (166 mg, 30 µmol) was reacted with the same amount of reagents as in example 5 to give **11** in a yield of 153 mg (90 %; capacity 0.08 mmol/g, coupling efficiency 44%).

### EXAMPLE 9

Resin **12** was prepared analogous to the synthesis of 9 described in Example 6.

NovaSyn TGR resin (166 mg, 30 µmol) was reacted with the same amount of reagents as in example 6 to give 12 in a yield of 142.2 mg (84%).

### EXAMPLE 10

In this example, a bidentate chelator is attached to Tentagel S AC bromide 1.

N,N'-Dimethylethylenediamine (110 µl, 1040 µmol) was dissolved in DMF (1 ml), resin **1** (108.6 mg, 26 µmol) was added, and the mixture was gently stirred at room temperature for 24 hours. The reaction mixture was filtered, and the resin was washed with DMF (3 times), DMF and methanol (3 times alternating), water (3 times), DMF and isopropanol (3 times alternating), water (3 times, iospropanol (3 times), and diethyl ether (3 times). The resin was dried at high vacuum to give product **13** in a yield of 101.6 mg (98.1%; capacity 0.24 mmol/g, coupling efficiency 100%). Resin 13 was positive on bromophenol blue and negative on TNBS staining.

### EXAMPLE 11

In this example, the synthesis of a conjugate yielding a bioactive complex, which intercalates into double stranded DNA is described. The bioactive unit, a pyrene derivative, is attached to the chelating unit on the solid support.

***N*-Boc-*N*''-Dde** protected diethylene triamine was coupled to **1** as described in the preparation of **3.** The bis-protected intermediate was positive on bromophenol blue and negative on TNBS staining. The Dde protecting group was removed by stirring the resin in hydrazine hydrate (1.5 ml, 2% in DMF) five times for 10 minutes, followed by filtration. Positive staining with TNBS confirmed the removal of the Dde protecting group. The pyrene group was introduced by reductive amination. 1-Pyrenaldehyde (28.5 mg, 120 µmol) and methanol (4 ml) were added to the mono-deprotected resin (103 mg, 24 µmol), and the mixture was stirred at room temperature for 20 hours. After filtration and washing with DMF, sodium triacetoxyborohydride (25 mg, 120 µmol) in DMF (5 ml) was added, and the mixture was stirred for **24** hours at room temperature. Washing with DMF and methanol (as above) gave the Boc-protected resin bound pyrene diethylenetriamine derivative which was positive on bromophenol blue and negative on TNBS staining. Finally, the Boc protecting group was removed by stirring the resin in trifluoroacetic acid (50% in CH₂Cl₂) for 5 minutes, followed by filtration and another treatment with trifluoroacetic acid (50% in CH₂Cl₂) for 10 minutes. Washing (as described above) gave product **7** (capacity 0.18 mmol/g, coupling efficiency 82%). Resin **7** was positive on bromophenol blue and on TNBS staining.

### EXAMPLE 12

In this example, biotin (Vitamin H) is attached to the chelating unit. In contrast to example 11, the chelator/biomolecule-conjugate is synthesized prior to the binding to the solid support.

Triethylenetetramine (0.150 ml, 1.00 mmol) was dissolved in THF (30 ml), and the solution was cooled to -78°C. A solution of ethyl trifluoroacetate (0.109 ml, 1.00 mmol) in THF (5 ml) was added within 30 min at -78°C, and the solution was stirred at that temperature for **4** hours. Then it was warmed up to 0°C.

In the meantime, (+)-Biotin (244 mg, 1.00 mmol) in DMF (8 ml) was heated to 80°C to give a colorless solution. N,N'-Dicyclohexylcarbodiimide (216 mg, 1.05 mmol) and N-hydroxysuccinimide (121 mg, 1.05 mmol) was added to the hot solution. The mixture was allowed to slowly cool down to room temperature. A white powder precipitated. Stirring was continued for 4 hours.

The two mixtures were mixed at 0°C and stirred for 30 minutes to give a white gel. The THF was evaporated to give a white suspension. After stirring at room temperature for 18 hours, the solvent was removed in vacuo and water was added to the residue. The pH was adjusted to 3-4. The mixture was filtered, the eluate was neutralized and the water was removed in vacuo. The crude product was purified by column chromatography (silica, dichloromethane/methanol/triethylamine 5:1:0.1) to give N-biotinyl-N'''-trifluoroacetyl-triethyltetramine in a yield of 60.5 mg (0.129 mmol, 12.9%). The structure was confirmed by mass spectroscopy and NMR.

N-Biotinyl-N'''-trifluoroacetyl-triethyltetramine (40.3 mg, 86 µmol), triethylamine (1.8 µl, 17 µmol) and resin **1** (71.7 mg, 17 µmol) were reacted as described in example 1. The TFA-resin was dried at high vacuum to give product **14** in an yield of 67 mg (85%, capacity 3 mmol/g, coupling efficiency 13%). Resin **14** was positive on bromophenol blue and negative on TNBS staining. Attempts to remove the TFA protecting group with sodium carbonate (10 % in water) failed according to negative results upon TNBS staining.

### EXAMPLE 13

In this example, a method for the preparation of labeled peptide derivatives is described. A protected dipeptide with a free carboxylic end group was coupled to a partially protected polyamine. All of the protecting groups were removed, and Dde protection was introduced to selectively block the primary amino groups at the peptide and the chelator. This allowed to selectively bind the conjugate to a solid support via formation of a tertiary amino group from one of the unprotected secondary amino groups of the chelator.

*N*,*N*',*N*''-tri(tert-butyloxycarbonyl)triethylenetetramine (103.3 mg, 234.5 µmol), Boc-Phe-Gly-OH (75.6 mg, 234.5 µmol), PyBOP (183 mg, 352 µmol) and diisopropyl ethylamine (20 µl, 117 µmol) were dissolved in dichloromethane (2.5 ml). The solution was stirred at room temperature for **4** hours. Periodically, diisopropylethylamine (20 µl, 117 µmol) was added to keep the pH >7. The solvent was removed *in vacuo*, the residue was dissolved in ethyl acetate and washed in brine (3 times), cold HCl 0.5M (3 times), NaHCO₃ 10% (2 times), and brine (3 times). The organic phase was dried over MgSO₄, and the solvent was removed in vacuo to give *N*-(*tert-*butyloxycarbonyl-phenylalanyl-glycyl)-*N*',*N*'',*N*'''-tri(*tert-*butyloxycarbonyl)-triethylenetetramine in an yield of 175.2 mg (231.6 µmol, 98.8%). The structure was confirmed by mass spectroscopy and NMR.

The Boc protecting groups were removed by stirring the product (162.2 mg, 0.169 mmol) in HCl in ethyl acetate (ca. 1 M, 3 ml). After stirring at room temperature for 9 hours, the solvent was removed in vacuo. The residue was dissolved in water and stirred for 1 hour (pH was 2-3), then NaOH (1 M, 313 mmol) was added to neutralize the solution, and the solvent was evaporated in vacuo to give H-Phe-Gly-NH-C₂H₄-NH-C₂H₄-NH-C₂H₄-NH₂ in quantitative yield. The structure was confirmed by mass spectroscopy and NMR.

H-Phe-Gly-NH-C₂H₄-NH-C₂H₄-NH-C₂H₄-NH₂ (113 mg, 0.152 mmol) and 2-Acetyldimedone (Dde-OH) (70.0 mg, 0.335 mmol) were dissolved in ethanol (4 ml). After stirring at room temperature for 20 hours, analysis by TLC exhibited full conversion of the amine to a single product. The solvent was removed to give Dde-Phe-Gly-NH-C₂H₄-NH-C₂H₄-NH-C₂H₄-NH-Dde. The structure was confirmed by mass spectroscopy. The crude product was used without separation from the surplus of 2-acetyldimedone.

Dde-Phe-Gly-NH-C₂H₄-NH-C₂H₄-NH-C₂H₄-NH-Dde (76 µmol) and resin 1 (79.2 mg, 19 µmol) were reacted as described in example 1. The protected intermediate was positive on bromophenol blue and negative on TNBS staining. The Dde protecting groups were removed as well as described in example 1, to give product **15.** Resin **15** was positive on bromophenol blue and on TNBS staining.

### EXAMPLE 14

Labeling conditions: [^{99m}Tc (H₂O)₃(CO)₃]⁺ was prepared out of [⁹⁹TcO₄]⁻ using a boroncarbonate kit (Alberto et al, J. Am. Chem. Soc. 123, 3135-3136 (2001)). 1 mg of the solid-phase bound chelators (0.2 mmole) were given to the [^{99m}Tc(H₂O)₃(CO)₃]⁺-solution (1 ml), the mixtures were shortly sonificated and then heated to 82°C for 30 minutes. The solutions were separated from the solid phase resin by filtration and analyzed by HPLC with gamma-detection.

With all of the solid phase bound chelators, formation of soluble complexes was observed. The yield varied on chelator type and reaction conditions between 5 to 50% (Table 1).

### EXAMPLE 15

Resins 3 and **4** were also labeled in a one pot procedure, combining the formation of [^{99m}Tc(H₂O)₃(CO)₃]⁺ and the cleavage reaction. 1 mg of solid-phase bound chelators (0.2 mmole) was added to a boroncarbonate kit (Mallinckrodt Medical, Petten, the Netherlands; Alberto et al., J. Am. Chem. Soc. 123, 3135-3136 (2001)). NaTcO₄ as eluted from a generator was added to the vial, and the mixture was kept at 78°C to 82°C for 20 to 60 minutes. The pH was 11. Cleavage yield was between 8 and 32%, conversion of pertechnetate between 40 and 54%.

### EXAMPLE 16

For the labeling reactions on resin 4, reaction conditions such as pH value and reaction temperature were varied to find optimal reaction conditions. For use as a radiotracer, complete conversion of the starting material [^{99m}Tc(H₂O)₃(CO)₃]⁺ and formation of one single product is required. Purification steps after the labeling procedure have to be avoided because of the radioactivity of the samples and their rapid decay (t_{1/2} = 6.2 hours). High cleavage yields are desirable to get solutions of high radioactivity.

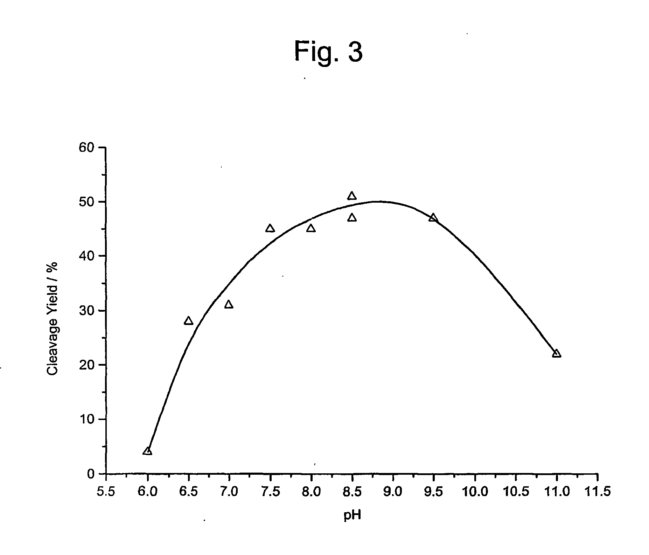

pH dependence of the cleavage reaction is shown in **Figure 3****.** Cleavage yield increases from pH 6 with a maximum at pH 8.5. This is in consistence with deprotonation of [^{99m}Tc(H₂O)₃(CO)₃]⁺ to [^{99m}Tc(OH)(H₂O)₂(CO)₃] (pKₛ for the Rhenium analog: 7.5; Egli et al, Organometallics 16, 1833-1840 (1997)) and, therefore, with the theory that a Tc-coordinated hydroxy ion is the nucleophile which attacks the CH₂-group to cleave the C-N bond. Increasing the pH to 11 reduces the cleavage yield again. This could be due to formation of the negatively charged species [^{99m}Tc(OH)₂(H₂O)(CO)₃]⁻ which reduces the electrophilicity of a coordinated amino group.

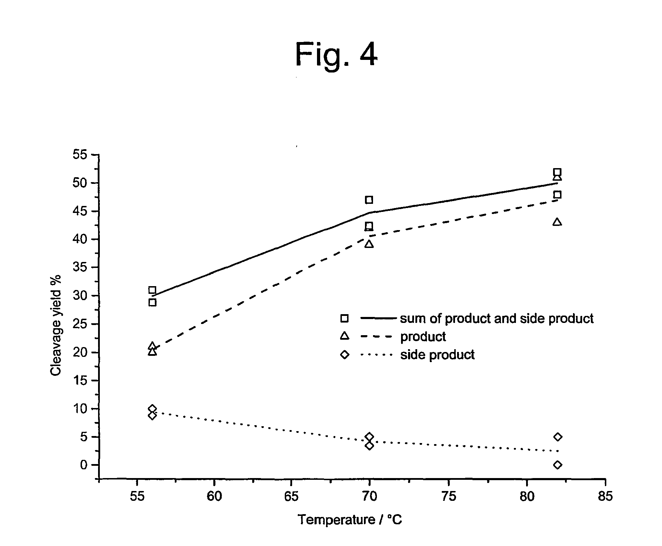

Temperature dependence of the reaction of resin **4** with [^{99m}Tc(H₂O)₃(CO)₃]⁺ was studied by analyzing the reaction products after full conversion of [^{99m}Tc(H₂O)₃(CO)₃]⁺. At room temperature, only complex formation at the resin occurred, the solution after filtration from the solid phase exhibited no radioactivity. At 43°C, cleavage yield was observable but low. Then, it increased with increasing temperature **(****Figure 4****).** This clearly shows that there is a competition between complex formation at the resin and the cleavage reaction, with the cleavage reaction having the higher activation energy barrier. However, very high temperatures could be a disadvantage in view of the stability of the solid phase resin and of attached biomolecules. A reaction temperature of 70°C to 82°C is preferred for resin **4.**

**Table 1**

| solid-phase bound chelator (resin) | cleaved ^{99m}Tc-complex | cleavage yield | conditions |
|---|---|---|---|
| 3 (TentaGel) | 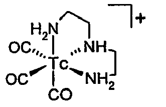 | 27% | 1 hour 82°C |
| 4 (TentaGel) | 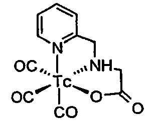 | 52% | 1 hour 82°C |
| | | 28% | 2 hours 56°C |
| 5 (TentaGel) | 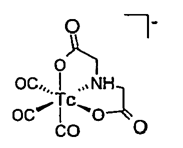 | 20% | 1 hour 82°C |
| 6 (TentaGel) | 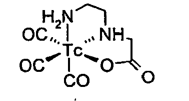 | 22% | 1 hour 82°C |
| 7 (TentaGel) | 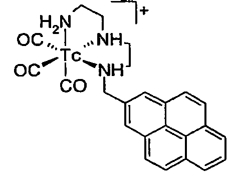 | 5% | 1 hour 82°C |
| 8 (NovaSyn TG) | 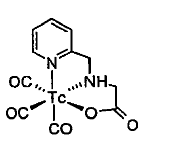 | 8% | 1 hour 82°C |
| 9 (NovaSyn TG) | 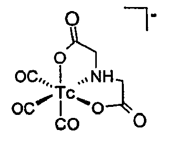 | 11% | 1 hour 82°C |
| | | 4% | 3 hours 56°C |
| 10 (NovaSyn TGT) | 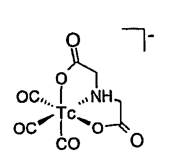 | 93% | 1 hour 82°C |
| | | 79% | 3 hours 56°C |
| 11 (NovaSyn TGR) | 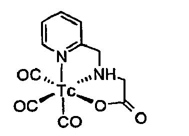 | 53% | 1 hour 82°C |
| | | 15% | 3 hours 56°C |
| 12 (NovaSyn TGR) | 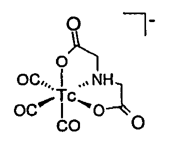 | 83% | 1 hour 82°C |
| | | 78% | 3 hours 56°C |
| 13 (TentaGel) | 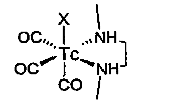 | 10% | 1 hour 82°C |
| 14 (TentaGel) | 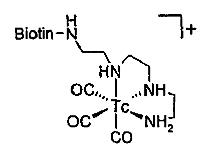 | 14% | 1 hour 82°C |
| 14 (TentaGel) | 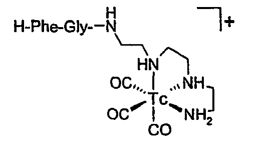 | 13% | 2 hours 82°C |
| | | | |

## Claims

1. Process for generating a metal complexed agent, comprising:
contacting a solid phase bound organic conjugate represented by formula (I) with [M(H₂O)₃(CO)₃]ⁿ⁺, 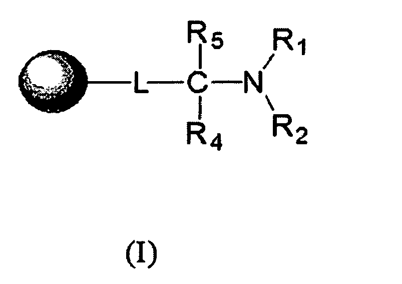
wherein:
the sphere is a solid phase support;
C is a methylene group;
R4 and R5 are independently selected from the group consisting of H, aliphatic substituents, aromatic substituents, RO, RS and (R)₂N, wherein R is an aliphatic or arylic group;
L is a linker or a single bond; and
each of R1 and R2 is independently a metal coordinating group, a non-coordinating organic group, a metal coordinating group derivatized with a biologically active molecule, or a non-coordinating organic group derivatized with a biologically active molecule,
wherein M is selected from the group consisting of technetium (Tc), rhenium (Re), rhodium (Rh), platinum (Pt), iridium (Ir), ruthenium (Ru), and copper (Cu); and
n is 1, 2 or 3.

2. Process according to claim 1, wherein L is a linker selected from the group consisting of phenylene, vinyl, alkylene, allylene, arylene, and other non-aliphatic and aliphatic groups.

3. Process according to claim 1 or 2, wherein L is substituted with an electron withdrawing group selected from OR, R and (R)₂N, wherein R is an aliphatic or arylic group.

4. Process according to any one of the claims 1-3,
wherein L is: 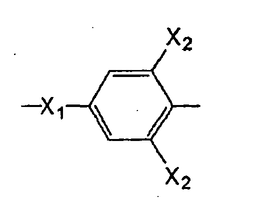 wherein X₁ is O, and each of X₂ is H or an electron withdrawing substituent, provided that at least one X₂ group is an electron withdrawing substituent.

5. Process according to any one of the claims 1-4,
wherein at least one of R1 and R2 is selected from the group consisting of 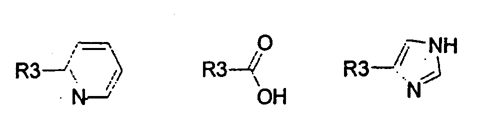
R3-NH₂
wherein R3 is an aliphatic chain containing 1, 2 or 3 carbons.

6. Process according to any one of the claims 1-5,
wherein at least one of R1 and R2 is an aliphatic or aromatic substituent.

7. Process according to any one of the claims 1-6, further comprising forming a coordinate bond between [M(H₂O)₃(CO)₃]ⁿ⁺ and a tertiary amine nitrogen atom of the solid phase bound organic conjugate, and releasing a metal complexed agent thus formed.

8. Process according to any one of the claims 1-7,
wherein M is selected from the group consisting of ^{99m}Tc, ¹⁸⁶Re and ¹⁸⁸Re.

9. Process according to any one of the claims 1-8,
wherein the biologically active molecule is selected from the group consisting of amino acids, steroids, peptides, proteins, carbohydrates, polysaccharides, oligosaccharides, nucleosides, nucleotides, oligonucleotides, polynucleotides, lipids, and pharmaceutically active small molecules.

10. Process according to any one of the claims 1-9,
wherein the solid phase support is a polyethylene glycol resin or a hybrid of polyethylene glycol and polystyrene.

11. Process according to any one of the claims 1-10,
wherein the process is performed at a pH that is in the range of about 6.0-11.0.

12. Process according to any one of the claims 1-11,
wherein the process is performed at a temperature in the range of about 40-100°C.

13. A solid phase bound organic conjugate represented by formula (I) 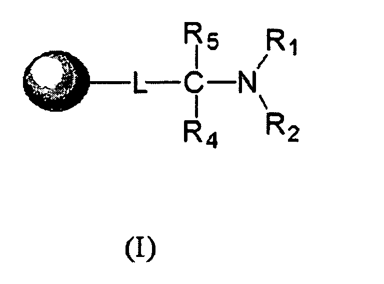 wherein the sphere is a solid phase support;
C is a methylene group;
R4 and R5 are independently selected from the group consisting of H, aliphatic substituents, aromatic substituents, RO, RS and (R)₂N, wherein R is an aliphatic or arylic group;
L is a linker or a single bond; and
each of R1 and R2 is independently a metal coordinating group, a non-coordinating organic group, a metal coordinating group derivatized with a biologically active molecule, or a non-coordinating organic group derivatized with a biologically active molecule.

14. A solid phase bound organic conjugate according to claim 13, wherein the biologically active molecule is selected from the group consisting of amino acids, steroids, peptides, proteins, carbohydrates, polysaccharides, oligosaccharides, nucleosides, nucleotides, oligonucleotides, polynucleotides, lipids, and pharmaceutically active small molecules.

15. A solid phase bound organic conjugate according to claim 13 or 14, wherein the solid phase support is a polyethylene glycol resin or a hybrid of polyethylene glycol and polystyrene.

16. A kit for the preparation of a diagnostic or therapeutic pharmaceutical composition, the kit comprising:
a container; and
a molecule of formula (I), 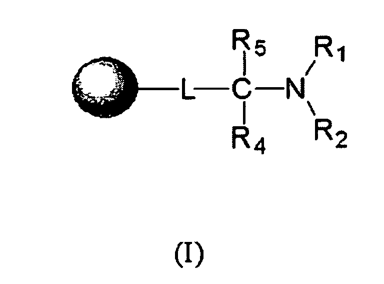
wherein the sphere is a solid phase;
C is a methylene group;
R4 and R5 are independently selected from the group consisting of H, aliphatic substituents, aromatic substituents, RO, RS and (R)₂N, wherein R is an aliphatic or arylic group;
L is a linker or a single bond; and
each of R1 and R2 is independently a metal coordinating group, a non-coordinating organic group, a metal coordinating group derivatized with a biologically active molecule, or a non-coordinating organic group derivatized with a biologically active molecule,
in which the reaction with a solution of [M(H₂O)₃(CO)₃]ⁿ⁺ can take place.

17. Kit as claimed in claim 16, wherein the container is a vessel or column.

18. Kit as claimed in claim 16 or 17, further comprising a solution of [M(H₂O)₃(CO)₃]ⁿ⁺, wherein M is a metal, and n is 1, 2 or 3.

19. Kit as claimed in any one of the claims 16-18, further comprising reagents for preparation of [M(H₂O)₃(CO)₃]ⁿ⁺,
wherein M is a metal, and n is 1, 2 or 3.

20. Kit as claimed in any one of the claims 16-19, further comprising a facility for filtration.

## Patentansprüche

1. Verfahren zum Herstellen eines Metall-komplexierten Stoffs, umfassend:
in Kontakt bringen eines an eine Festphase gebundenen organischen Konjugats, das durch eine Formel (I) mit [M(H₂O)₃(CO)₃]ⁿ⁺, 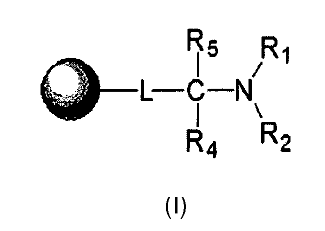
dargestellt ist, wobei:
die Kugel ein Festphasenträger ist;
C eine Methylengruppe ist;
R4 und R5 unabhängig aus der Gruppe ausgewählt sind, bestehend aus H, aliphatischen Substituenten, aromatischen Substituenten, RO, RS und (R)₂N, wobei R eine aliphatische oder Arylgruppe ist;
L ein Linker oder ein Einfachbindung ist; und
jeder von R1 und R2 unabhängig eine Metallkoordinierungsgruppe, eine organische nicht-Koordinierungsgruppe, eine Metallkoordinierungsgruppe, die mit einem biologisch aktiven Molekül derivatisiert ist, oder eine organische nicht-Koordinierungsgruppe ist, die mit einem biologisch aktiven Molekül derivatisiert ist,
wobei M aus der Gruppe ausgewählt ist, bestehend aus Technetium (Tc), Rhenium (Re), Rhodium (Rh), Platin (Pt), Iridium (Ir), Ruthenium (Ru) und Kupfer (Cu); und
n 1, 2 oder 3 ist.

2. Verfahren gemäß Anspruch 1, wobei L ein Linker ist, der aus der Gruppe ausgewählt ist, bestehend aus Phenylen, Vinyl, Alkylen, Allylen, Arylen und anderen nicht-aliphatischen und aliphatischen Gruppen.

3. Verfahren gemäß Anspruch 1 oder 2, wobei L mit einer Elektron abziehenden Gruppe substituiert ist, die aus OR, R und (R)₂N ausgewählt ist, wobei R eine aliphatische oder Arylgruppe ist.

4. Verfahren gemäß einem der Ansprüche 1 - 3, wobei L 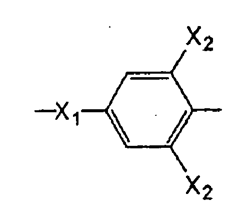 ist, wobei X₁ O ist und jeder von X₂ H oder ein Elektron abziehender Substituent ist, vorausgesetzt, dass mindestens eine X₂-Gruppe ein Elektron abziehender Substituent ist.

5. Verfahren gemäß einem der Ansprüche 1 - 4, wobei mindestens einer von R1 und R2 aus der Gruppe ausgewählt ist, bestehend aus 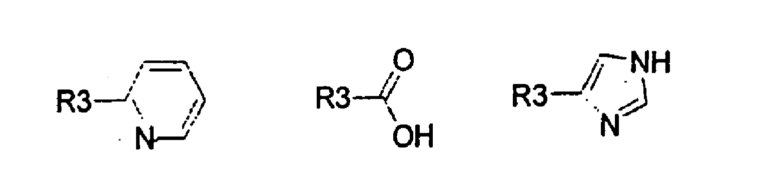
R3-NH₂
wobei R3 eine aliphatische Kette ist, die 1, 2 oder 3 Kohlenstoffe enthält.

6. Verfahren gemäß einem der Ansprüche 1 - 5, wobei mindestens einer von R1 und R2 ein aliphatischer oder aromatischer Substituent ist.

7. Verfahren gemäß einem der Ansprüche 1 - 6, weiter umfassend Bilden einer Koordinatenbindung zwischen [M(H₂O)₃ (CO)₃]ⁿ⁺ und einem tertiären AminStickstoffatom des an die Festphase gebundenen organischen Konjugats, und Freisetzen eines gebildeten Metall-komplexierten Stoffs.

8. Verfahren gemäß einem der Ansprüche 1 - 7, wobei M aus der Gruppe ausgewählt ist, bestehend aus ^{99m}Tc, ¹⁸⁶Re und ¹⁸⁸Re.

9. Verfahren gemäß einem der Ansprüche 1 - 8, wobei das biologisch aktive Molekül aus der Gruppe ausgewählt ist, bestehend aus Aminosäuren, Steroiden, Peptiden, Proteinen, Kohlenhydraten, Polysacchariden, Oligosacchariden, Nukleosiden, Nukleotiden, Oligonukleotiden, Polynukleotiden, Lipiden und pharmazeutisch aktiven kleinen Molekülen.

10. Verfahren gemäß einem der Ansprüche 1 - 9, wobei der Festphasenträger ein Polyethylenglykol Harz oder ein Hybrid aus Polyethylenglykol und Polystyrol ist.

11. Verfahren gemäß einem der Ansprüche 1 - 10, wobei das Verfahren bei einem pH-Wert durchgeführt wird, der im Bereich von etwa 6,0 - 11,0 liegt.

12. Verfahren gemäß einem der Ansprüche 1 - 11, wobei das Verfahren bei einer Temperatur im Bereich von etwas 40 - 100 °C durchgeführt wird.

13. An eine Festphase gebundenes organisches Konjugat, das durch eine Formel (I) 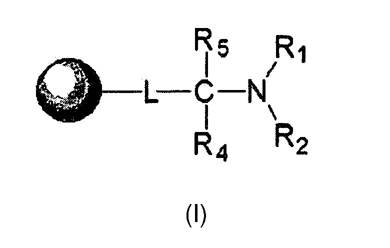 dargestellt ist, wobei die Kugel ein Festphasenträger ist;
C eine Methylengruppe ist;
R4 und R5 unabhängig aus der Gruppe ausgewählt sind, bestehend aus H, aliphatischen Substituenten, aromatischen Substituenten, RO, RS und (R)₂N, wobei R eine aliphatische oder Arylgruppe ist;
L ein Linker oder eine Einfachbindung ist; und
jeder von R1 und R2 unabhängig eine Metallkoordinierungsgruppe, eine organische nicht-Koordinierungsgruppe, eine Metallkoordinierungsgruppe, die mit einem biologisch aktiven Molekül derivatisiert ist, oder eine organische nicht-Koordinierungsgruppe ist, die mit einem biologisch aktiven Molekül derivatisiert ist.

14. An eine Festphase gebundenes organisches Konjugat gemäß Anspruch 13, wobei das biologisch aktive Molekül aus der Gruppe ausgewählt ist, bestehend aus Aminosäuren, Steroiden, Peptiden, Proteinen, Kohlenhydraten, Polysachariden, Oligosacchariden, Nukleosiden, Nukleotiden, Oligonukleotiden, Polynukleotiden, Lipiden und pharmazeutisch aktiven kleinen Molekülen.

15. An eine Festphase gebundenes organisches Konjugat gemäß Anspruch 13 oder 14, wobei der Festphasenträger ein Polyethylenglykol Harz oder ein Hybrid aus Polyethylenglykol und Polystyrol ist.

16. Kit zur Herstellung einer diagnostischen oder therapeutischen pharmazeutischen Zusammensetzung, der Kit umfasst:
einen Behälter; und
ein Molekül der Formel (I), 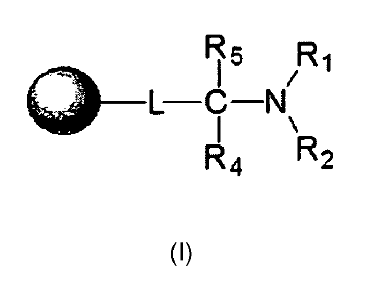
wobei die Kugel eine Festphase ist;
C eine Methylengruppe ist;
R4 und R5 unabhängig aus der Gruppe ausgewählt sind, bestehend aus, H, aliphatischen Substituenten, aromatischen Substituenten, RO, RS und (R)₂N, wobei R eine aliphatische oder Arylgruppe ist;
L ein Linker oder eine Einfachbindung ist; und
jeder von R1 und R2 unabhängig eine Metallkoordinierungsgruppe, eine organische nicht-Koordinierungsgruppe, eine Metallkoordinierungsgruppe, die mit einem biologisch aktiven Molekül derivatisiert ist, oder eine organische nicht-Koordinierungsgruppe ist, die mit einem biologisch aktiven Molekül derivatisiert ist,
in welchem die Reaktion mit einer Lösung aus [M(H₂O)₃ (CO)₃]ⁿ⁺ stattfinden kann.

17. Kit wie in Anspruch 16 beansprucht, wobei der Behälter ein Gefäß oder eine Säule ist.

18. Kit wie in Anspruch 16 oder 17 beansprucht, weiter umfassend eine Lösung aus [M(H₂O)₃ (CO)₃]ⁿ⁺, wobei M ein Metall ist und n 1, 2 oder 3 ist.

19. Kit wie in einem der Ansprüche 16 - 18 beansprucht, weiter umfassend Reagenzien zur Herstellung von [M(H₂O)₃ (CO)₃]ⁿ⁺, wobei M ein Metall ist und n 1, 2 oder 3 ist.

20. Kit wie in einem der Ansprüche 16 - 19 beansprucht, weiter umfassend eine Einrichtung zur Filtration.

## Revendications

1. Procédé de génération d'un agent complexé métallique, comportant l'étape consistant à :
mettre en contact un conjugué organique lié à une phase solide représenté par la formule (I) avec [M(H₂O)₃(CO)₃]ⁿ⁺, 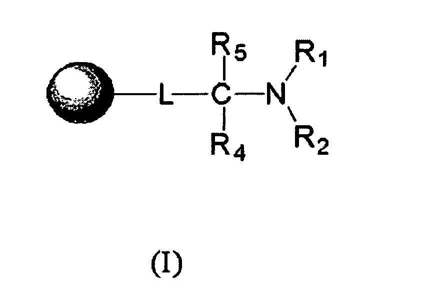
dans laquelle :
la sphère est un support de phase solide,
C est un groupe méthylène,
R₄ et R₅ sont sélectionnés indépendamment dans le groupe comprenant H, des substituants aliphatiques, des substituants aromatiques, RO, RS et (R)₂N, R étant un groupe aliphatique ou aryle,
L est un bras espaceur ou une liaison simple, et
R₁ et R₂ sont chacun indépendamment un groupe de coordination métallique, un groupe de non-coordination organique, un groupe de coordination métallique dérivé avec une molécule biologiquement active, ou un groupe de non-coordination organique dérivé avec une molécule biologiquement active,
dans lequel M est choisi dans le groupe comprenant du technétium (Tc), du rhénium (Re), du rhodium (Rh), du platine (Pt), de l'iridium (Ir), du ruthénium (Ru), et du cuivre (Cu), et
n est égal à 1, 2 ou 3.

2. Procédé selon la revendication 1, dans lequel L est un bras espaceur sélectionné dans le groupe comprenant des groupes phénylène, vinyle, alkylène, allylène, arylène, et d'autres groupes aliphatiques et non aliphatiques.

3. Procédé selon la revendication 1 ou 2, dans lequel L est substitué par un groupe accepteur d'électrons sélectionné parmi OR, R et (R)₂N, dans lequel R est un groupe aliphatique ou aryle.

4. Procédé selon l'une quelconque des revendications 1 à 3, dans lequel L est : 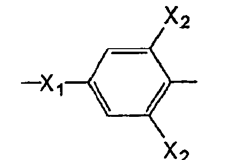 dans lequel X₁ est O, et chacun des X₂ est H ou un substituant accepteur d'électrons, pourvu qu'au moins un groupe X₂ soit un substituant accepteur d'électrons.

5. Procédé selon l'une quelconque des revendications 1 à 4, dans lequel au moins un d'entre R1 et R2 est sélectionné dans le groupe comprenant 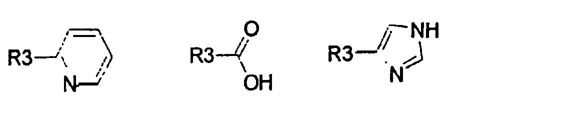
R3-NH₂
dans lequel R3 est une chaîne aliphatique contenant 1, 2 ou 3 atomes de carbone.

6. Procédé selon l'une quelconque des revendications 1 à 5, dans lequel au moins un d'entre R1 et R2 est un substituant aliphatique ou aromatique.

7. Procédé selon l'une quelconque des revendications 1 à 6, comportant en outre la formation d'une liaison coordonnée entre [M(H₂O)₃(CO)₃]ⁿ⁺ et un atome d'azote d'amine tertiaire du conjugué organique lié à une phase solide, et la libération d'un agent complexé métallique ainsi formé.

8. Procédé selon l'une quelconque des revendications 1 à 7, dans lequel M est sélectionné dans le groupe comprenant ^{99m}Tc, ¹⁸⁶Re et ¹⁸⁸Re.

9. Procédé selon l'une quelconque des revendications 1 à 8, dans lequel la molécule biologiquement active est sélectionnée dans le groupe constitué d'acides aminés, de stéroïdes, de peptides, de protéines, de carbohydrates, de polysaccharides, d'oligosaccharides, de nucléosides, de nucléotides, d'oligonucléotides, de polynucléotides, de lipides, et de petites molécules pharmaceutiquement actives.

10. Procédé selon l'une quelconque des revendications 1 à 9, dans lequel le support de phase solide est une résine de polyéthylèneglycol ou un hydride de polyéthylèneglycol et de polystyrène.

11. Procédé selon l'une quelconque des revendications 1 à 10, dans lequel le procédé est mis en oeuvre à un pH qui est situé dans la plage d'environ 6,0 à 11,0.

12. Procédé selon l'une quelconque des revendications 1 à 11, dans lequel le procédé est mis en oeuvre à une température située dans la plage d'environ 40°C à 100°C.

13. Conjugué organique lié à une phase solide représenté par la formule (I) : 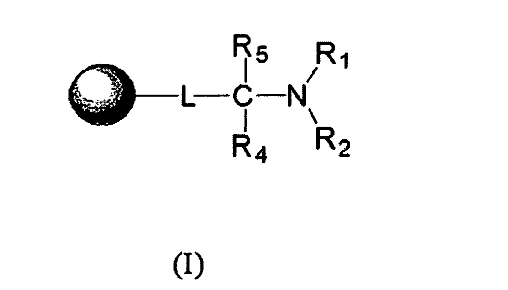 dans laquelle la sphère est un support de phase solide,
C est un groupe méthylène,
R₄ et R₅ sont sélectionnés de manière indépendante dans le groupe comprenant H, des substituants aliphatiques, des substituants aromatiques, RO, RS, et (R)₂N, R étant un groupe aliphatique ou aryle,
L est un bras espaceur ou une liaison simple, et
R₁ et R₂ sont chacun de manière indépendante un groupe de coordination métallique, un groupe de non-coordination organique, un groupe de coordination métallique dérivé avec une molécule biologiquement active, ou un groupe de non-coordination organique dérivé avec une molécule biologiquement active.

14. Conjugué organique lié en phase solide selon la revendication 13, dans lequel la molécule biologiquement active est sélectionnée dans le groupe constitué d'acides aminés, de stéroïdes, de peptides, de protéines, de carbohydrate, de polysaccharides, d'oligosaccharides, de nucléosides, de nucléotides, d'oligonucléotides, de polynucléotides, de lipides, et de petites molécules pharmaceutiquement actives.

15. Conjugué organique lié à une phase solide selon la revendication 13 ou 14, dans lequel le support de phase solide est une résine de polyéthylèneglycol ou un hybride de polyéthylèneglycol et de polystyrène.

16. Kit de préparation d'une composition pharmaceutique de diagnostic ou thérapeutique, le kit comprenant :
un conteneur, et
une molécule selon la formule (I), 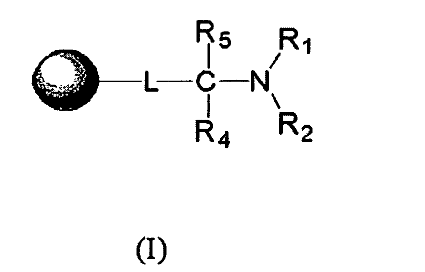
dans laquelle la sphère est une phase solide,
C est un groupe méthylène,
R₄ et R₅ sont sélectionnés de manière indépendante dans le groupe comprenant H, des substituants aliphatiques, des substituants aromatiques, RO, RS et (R)₂N, R étant un groupe aliphatique ou aryle,
L est un bras espaceur ou une liaison simple, et
R₁ et R₂ sont de manière indépendante un groupe de coordination métallique, un groupe de non-coordination organique, un groupe de coordination métallique dérivé avec une molécule biologiquement active, ou un groupe de non-coordination organique dérivé avec une molécule biologiquement active,
dans lequel la réaction avec une solution de [M(H₂O)₃(CO)₃]ⁿ⁺ peut intervenir.

17. Kit selon la revendication 16, dans lequel le conteneur est un récipient ou une colonne.

18. Kit selon la revendication 16 ou 17, comportant en plus une solution de [M(H₂O)₃(CO)₃]ⁿ⁺, dans lequel M est un métal, et n est égal à 1, 2 ou 3.

19. Kit selon l'une quelconque des revendications 16 à 18, comportant en outre des réactifs pour la préparation de [M(H₂O)₃(CO)₃]ⁿ⁺, dans lequel M est un métal, et n est égal à 1, 2 ou 3.

20. Kit selon l'une quelconque des revendications 16 à 19, comportant en outre un dispositif de filtrage.
